# EUROPÄISCHE PATENTSCHRIFT

(11) **EP 3 941 732 B1**
(45) Veröffentlichungstag und Bekanntmachung des Hinweises auf die Patenterteilung: **11.10.2023**
(21) Anmeldenummer: 20707029.3
(22) Anmeldetag: 19.02.2020
(51) Int. Cl.: B30B 15/00, B30B 5/06, G01N 33/28, G01N 21/64, B27N 3/24, B27N 3/02, G01N 21/55, G01N 21/84, G01N 21/89, G01N 21/94, G01B 7/06, G01B 11/06, G01B 15/02, G01N 27/22

(54) **VORRICHTUNG UND VERFAHREN ZUR ÜBERWACHUNG DES SCHMIERZUSTANDES EINES MIT EINEM SCHMIERMITTEL BEAUFSCHLAGTEN UMLAUFENDEN BANDES FÜR DEN TRANSPORT VON PRESSGUT**
APPARATUS AND METHOD FOR MONITORING THE LUBRICATING STATE OF A ROTATING BELT FOR TRANSPORTING MATERIAL TO BE PRESSED, TO WHICH BELT A LUBRICANT HAS BEEN APPLIED
DISPOSITIF ET PROCÉDÉ DE SURVEILLANCE DE L'ÉTAT DE LUBRIFICATION D'UNE BANDE ROTATIVE POUR LE TRANSPORT D'UNE MATIÈRE À PRESSER, LA BANDE ÉTANT SOUMISE À L'EFFET D'UN LUBRIFIANT

(30) Priorität: 20.03.2019 DE 102019107152
(43) Veröffentlichungstag der Anmeldung: 26.01.2022
(60) Teilanmeldung: 23191073.8
(73) Patentinhaber: Siempelkamp Maschinen- und Anlagenbau GmbH, 47803 Krefeld (DE)
(72) Erfinder: GARTZ, Klaus, 41749 Viersen (DE)
(74) Vertreter: Andrejewski - Honke Patent- und Rechtsanwälte Partnerschaft mbB
(86) Internationale Anmeldenummer: PCT/EP2020/054336
(87) Internationale Veröffentlichungsnummer: WO 2020/187521

(56) Entgegenhaltungen:
- EP-A1- 0 585 589
- EP-A1- 1 914 538
- WO-A1-2016/037735
- DE-A1-102010 014 952
- DE-A1-102015 007 054
- DE-A1-102016 102 931
- DE-B4-102016 102 931
- JP-A- H03 252 512
- US-A- 4 892 133

## Beschreibung

Die Erfindung betrifft eine Vorrichtung zur Überwachung des Schmierzustandes eines mit einem Schmiermittel beaufschlagten, umlaufenden Bandes für den Transport von Pressgut, insbesondere in einer kontinuierlich arbeitenden bzw. kontinuierlich betreibbaren Presse, mit zumindest einer im Bereich des Bandes angeordneten ersten Detektionseinrichtung eines ersten Typs und mit zumindest einer im Bereich des Bandes angeordneten zweiten Detektionseinrichtung eines zweiten Typs.

Die Erfindung betrifft auch ein Verfahren zur Überwachung eines mit einem Schmiermittel beaufschlagten, umlaufenden Bandes für den Transport von Pressgut, insbesondere in einer kontinuierlich arbeitenden Presse, wobei mit zumindest einer im Bereich des Bandes angeordneten ersten Detektionsvorrichtung eines ersten Typs und mit zumindest einer im Bereich des Bandes angeordneten zweiten Detektionsvorrichtung eines zweiten Typs zumindest ein Signal erzeugt wird.

Außerdem betrifft die Erfindung eine kontinuierliche Presse, mit einem oberen, endlos umlaufenden Band bzw. Pressband und einem unteren endlos um laufenden Band bzw. Pressband, wobei Pressgut durch den Pressspalt zwischen dem oberen und dem unteren Band hindurchgeführt und unter Anwendung von Druck und/oder Wärme zu einer Platte bzw. einem endlosen Plattenstrang verpresst wird. Die endlos umlaufenden Bänder einer solchen Presse werden auch als Pressbänder bezeichnet und sie sind bevorzugt als Stahlbänder ausgebildet.

Schließlich betrifft die Erfindung ein Verfahren zum Betreiben einer kontinuierlich arbeitenden Presse.

Bevorzugt handelt es sich bei einer solchen kontinuierlich betreibbaren Presse um eine Doppelbandpresse, bei der im Pressenoberteil eine obere (beheizbare) Pressenplatte und im Pressenunterteil eine untere (beheizbare) Pressenplatte vorgesehen sind und wobei im Pressenoberteil das endlos umlaufende obere Pressband und im Pressenunterteil das endlos umlaufende untere Pressband geführt sind. Die Pressbänder sind bevorzugt unter Zwischenschaltung von Wälzkörpern, z. B. Rollstäben oder Rollstangen, an den Pressenplatten abgestützt. Die erforderlichen Presskräfte werden mit Kraftmitteln, insbesondere mit hydraulischen Presszylindern aufgebracht, mit denen z. B. die obere und/oder die untere Pressenplatte beaufschlagt wird und die sich an einem Pressengestell, z. B. an den Pressenrahmen eines Pressengestells abstützen. Die Rollstäbe sind z. B. an Rollstabketten angeschlossen und mit Hilfe solcher Rollstabketten im Umlauf geführt.

Eine solche Presse, die auch kontinuierlich arbeitende Presse genannt wird, wird z. B. für die Herstellung von Holzwerkstoffplatten eingesetzt. Holzwerkstoffplatten meint insbesondere Faserplatten, Spanplatten oder OSB-Platten. Alternativ betrifft die Erfindung aber auch derartige Pressen für die Herstellung von Verbundplatten oder von Verbundbauteilen, z. B. aus Faserverbundwerkstoffen oder dergleichen oder auch von Kunststoffplatten oder Kunststoffmatten.

Das Pressgut (z. B. eine Pressgutmatte) wird im Pressspalt der kontinuierlich arbeitenden Presse zwischen den umlaufenden Stahlbändern bzw. Pressbändern unter Anwendung von Druck- und Wärme verpresst, so dass aus dem Pressgut z. B. ein plattenförmiges Produkt, z. B. eine Holzwerkstoffplatte hergestellt wird. Dabei rollen die Rollstäbe an den Pressenplatten als Wälzkörperaggregate ab. Aufgrund üblicher Fertigungstoleranzen bzw. minimalsten Abweichungen der Rollstabform von der perfekten Zylinderform kann es im Zuge der Abrollbewegung zu Weg- bzw. Längenunterschieden an den beiden äußeren Enden der Rollstäbe kommen, die durch einen Mikroschlupf kompensiert werden. Vor diesem Hintergrund ist es in der Praxis üblich und technisch erforderlich, die Rollstäbe bzw. deren zylindrische Oberfläche und/oder die korrespondierende Fläche des umlaufenden Bandes mit einem Schmiermittel zu schmieren. Ein solches Schmiermittel ist bevorzugt als flüssiges Schmiermittel (z. B. Öl) ausgebildet und es wird in der Praxis z. B. mit Hilfe von Düsen oder dergleichen im Einlaufbereich oder im Rollstabumlauf auf die Rollstäbe und/oder auf die Pressbänder aufgebracht, z. B. aufgesprüht. Es erfolgt demnach eine Beaufschlagung der den Rollstangen zugewandten (inneren) Oberfläche des endlos umlaufenden Bandes mit einem Schmiermittel, und zwar entweder unmittelbar durch z. B. Bedüsung der Bandoberfläche oder mittelbar über die mit dem Schmiermittel beaufschlagten (z. B. bedüsten) Rollstangen.

Für den einwandfreien Betrieb einer kontinuierlich betreibbaren Presse ist eine einwandfreie Schmierung von besonderer Bedeutung, wobei in der Regel einerseits eine Unterschmierung und andererseits eine Überschmierung verhindert werden soll. Es ist daher in der Praxis vorgesehen, den Schmierzustand bzw. die durch die Bedüsung aufgebrachte Schmiermittel- bzw. Ölmenge zu überwachen, und zwar in der Regel manuell bzw. visuell durch das Servicepersonal aufgrund von Erfahrungswerten. Dabei soll darauf geachtet werden, dass sich die auf die Bandoberfläche zur Schmierung aufgebrachte Schmiermittelmenge gleichmäßig verteilt und keine Bereiche entstehen, in denen die Schmierung aufgrund von Schmiermittelmangel, z. B. Ölmangel nicht ausreichend bemessen ist. Grundsätzlich haben sich diese einfachen Maßnahmen in der Praxis bewährt. Es besteht jedoch das Bedürfnis die Überwachung zu optimieren und insbesondere zu automatisieren. So besteht insbesondere das Bedürfnis, eine zuverlässige und quantifizierbare Überwachung einzurichten, um einerseits zuverlässig Mangelschmierungen zu verhindern und andererseits den Schmiermittelverbrauch z. B. aus wirtschaftlichen Gründen innerhalb der erforderlichen Grenzen zu halten.

Kontinuierlich arbeitende Pressen mit zugehörigen Schmiersystemen sind z. B. aus der DE 31 48 412 A1, DE 40 15 706 A1 sowie DE 41 26 717 C1 bekannt.

Aus der DE 10 2016 102 931 B4 sind ferner eine Vorrichtung zur Überwachung und/oder Regelung eines Schmierzustandes in einer kontinuierlich arbeitenden Presse gemäß dem Oberbegriff des Vorrichtungsanspruchs 1 und ein Verfahren gemäß dem Oberbegriff des Anspruchs 9 bekannt. Bei dieser Vorrichtung sind ein oder mehrere Sensoren zum Messen eins physikalischen Phänomens vorgesehen, das mit einer Schmiermittelmenge auf dem Band zusammenhängt. Dazu soll eine Auswerteeinheit zum Ermitteln eines Schmierparameters als Kenngröße für eine Schmiermittelmenge auf dem Band basierend auf dem Messwert des Sensors vorgesehen sein. Als Sensor kann z. B. ein Lichtsensor zum Messen eines Lichtreflexionswertes des Bandes und/oder einer Rollstange oder ein Lichtsensor zum Messen eines Lichtabsorptionswertes des Bandes und/oder einer Rollstange der Presse sein. Alternativ können ein Leitfähigkeitssensor, ein Laserabstandsensor oder ein Ultraschallsensor zum Einsatz kommen. Des Weiteren werden Druck-, Zug-, Leistungs- und/oder Kraftsensoren erwähnt. Es soll eine steuerbare Schmiereinheit zum Aufbringen von Schmiermittel vorgesehen sein, die mit einer Regeleinrichtung zur Regelung einer von der Schmiereinheit aufgebrachten Schmiermittelmenge basierend auf dem ermittelten Schmierparameter eingerichtet ist. Im Übrigen soll die Möglichkeit bestehen, Signale mehrerer gleicher oder verschiedener Sensoren zu kombinieren.

Im Übrigen kennt man aus der WO 2016/037735 A1 ein Verfahren zur Ermittlung des Zustandes einer Transportbandoberfläche an einem bewegten Transportband mithilfe einer Polarisationskamera. Mit dem Verfahren soll es möglich sein, den Oberflächenzustand eines Transportbandes aufgrund des in den Polarisationsebenen auftretenden Kontrastes zwischen den einzelnen unterschiedlich beanspruchten Transportbandabschnitten zu ermitteln. So sollen Wartung-, Pflege- oder auch Reparaturmaßnahmen am Transportband durchgeführt werden, sobald vollflächige oder abschnittsweise Veränderungen des Oberflächenzustandes ermittelt werden.

In der DE 10 2015 007 054 A1 wird im Übrigen ein Verfahren und eine Vorrichtung zur Bestimmung der Dicke von dünnen organischen Schichten beschrieben, z. B. bei der Produktion von Bandstahl oder Aluminium. Im Vordergrund steht die Überwachung einer Beölung von Metallbändern bei der Produktion solcher Metallbänder. Dazu soll die Bandoberfläche einerseits mit UV-Strahlung und andererseits mit Infrarotstrahlung bestrahlt und das reflektierte UV-Licht einerseits und das Infrarotlicht andererseits analysiert werden.

Die EP 0 585 589 A1 beschreibt ein Verfahren zur selbsttätigen, iterativen Prozessoptimierung von Ziehvorgängen in Pressen. Dabei wird auch die Schmierung der Platinen, die in der Presse umgeformt werden, für ein gleichbleibendes Ziehergebnis thematisiert, wobei die Schmierfilmdicke interessieren soll, die mittels eines beispielsweise kapazitiv arbeitenden Sensors gemessen werden kann.

Schließlich beschreibt die WO 99/31459 A1 ein Verfahren und eine Vorrichtung zur berührungslosen Feststellung oder Überprüfung eines Fluidauftrages, insbesondere eines Leim-, Kleber-, Lack- oder Kunststoffauftrages, auf einer Oberfläche eines Werkstückes, wobei sowohl vor als auch nach einer Fluidauftragseinheit zumindest ein Messsensor mit zumindest einer Elektrode angeordnet ist. Die Messsensoren sind als kapazitive Messsensoren ausgebildet.

Ausgehend von dem vorbekannten Stand der Technik liegt der Erfindung das technische Problem zugrunde, eine Vorrichtung sowie ein Verfahren zu schaffen, mit der bzw. mit dem sich der Schmierzustand eines umlaufenden Bandes, insbesondere in einer kontinuierlich arbeitenden Presse, in wirtschaftlicher Weise besonders zuverlässig überwachen lässt.

Zur Lösung dieser Aufgabe lehrt die Erfindung eine Vorrichtung mit den Merkmalen des Anspruchs 1 und ein Verfahren mit den Merkmalen des Anspruchs 9. Bei einer erfindungsgemäßen Vorrichtung ist vorgesehen, dass einerseits die erste Detektionseinrichtung dazu eingerichtet ist, ein von der Menge des auf dem Band befindlichen Materials abhängiges Mengensignal zu erzeugen und dass andererseits zur Unterscheidung eines auf dem Band befindlichen Schmiermittels von auf dem Band befindlichen Holzpartikeln oder von einem mit Holzpartikeln verunreinigten Schmiermittel die zweite Detektionseinrichtung dazu eingerichtet ist, ein von der Art des auf dem Band befindlichen Materials abhängiges Materialsignal zu erzeugen.

Die Erfindung geht dabei von der Erkenntnis aus, dass eine besonders zuverlässige Überprüfung bzw. Überwachung des Schmierzustandes eines umlaufenden Bandes durch Einsatz mehrerer Detektionseinrichtungen mit unterschiedlichen Detektionscharakteristiken möglich ist, indem nämlich die erste Detektionseinrichtung auf die mengenmäßige bzw. mengenabhängige Erfassung des Schmiermittels ausgerichtet ist und die zweite Detektionseinrichtung materialsensitiv arbeitet und der Unterscheidung verschiedener Materialien dient. Im Vordergrund steht dabei die Unterscheidung eines sauberen Schmiermittels (z. B. Öl) und folglich eines Schmiermittels ohne übermäßige Verschmutzung durch Holzpartikel von einem übermäßig verschmutzten Schmiermittel oder auch von Holzpartikeln selbst.

Zugleich soll die Erfindung jedoch auch eine mengenmäßige Erfassung des Schmiermittels und folglich eine mengenmäßige Überprüfung des Schmierzustandes ermöglichen, so dass z. B. eine Mangelschmierung einerseits und auch eine (unwirtschaftliche) Überschmierung andererseits quantitativ bestimmt oder zumindest erkannt werden kann. Die Ermittlung bzw. Angabe einer Materialmenge als Messsignal muss dabei nicht eine tatsächliche, absolute Mengenangabe sein, sondern das Mengensignal kann sich auf eine relative Angabe bzw. Information beschränken, z. B. derart, dass angegeben wird, ob eine Schmiermittelmenge innerhalb eines vorgegebenen Bereiches liegt oder nicht. Von besonderer Bedeutung ist die Tatsache, dass mit der ersten Detektionseinrichtung zuverlässig eine quantitative Analyse bzw. Aussage und folglich eine Bestimmung der Menge des auf dem Band befindlichen Materials möglich ist, ohne dass mit dieser Detektionseinrichtung Rückschlüsse auf das auf dem Band befindliche Material möglich sein müssen. Insofern kann die erste Detektionseinrichtung maßgeblich auf eine besonders zuverlässige und/oder einfache mengenmäßige bzw. mengenabhängige Erfassung des Schmiermittels ausgelegt sein, ohne dass dabei der Verschmutzungsgrad des Schmiermittels erkannt werden muss. Demgegenüber kann die zweite Detektionseinrichtung auf eine optimale Materialunterscheidung ausgerichtet sein, ohne dass mit der zweiten Detektionseinrichtung bzw. dem eigesetzten Verfahren eine Aussage über die Menge möglich ist bzw. möglich sein muss.

Erfindungsgemäß kann die Überprüfung des Schmierzustandes folglich automatisiert oder zumindest maschinell unterstützt werden, so dass keine subjektive Beurteilung durch einen Operateur erfolgen muss, und zwar weder bezogen auf die Schmiermittelmenge noch bezogen auf die Schmiermittelqualität. Denn die erfindungsgemäße Vorrichtung kann sowohl ein (elektronisches) Mengensignal erzeugen als auch ein (elektronisches) Materialsignal, die z. B. in geeigneten Melde- und/oder Rechnersystemen visualisierbar und gegebenenfalls auch weiterverarbeitbar sind, z. B. im Rahmen einer Steuerung der Presse und insbesondere im Rahmen der Steuerung des Schmiermittelauftrags. So kann die Vorrichtung ein Melde- und/oder Rechnersystem aufweisen, mit dem das Mengensignal und/oder das Materialsignal anzeigbar sind, und zwar akustisch und/oder optisch. Alternativ oder ergänzend kann mit dem Melde- und/oder Rechnersystem das Mengensignal und das Materialsignal (gemeinsam) derart ausgewertet werden, dass aus dem Materialsignal die Art des auf dem Band befindlichen Materials ermittelt werden kann. Insofern kann es sich zum einen um ein Meldesystem handeln, das insbesondere der Visualisierung der bereits vorliegenden Signale dient. Alternativ kann in diesem System jedoch auch eine Auswertung der Signale zum Zwecke der Unterscheidung unterschiedlicher Materialien erfolgen.

Die erste Detektionseinrichtung, die der quantitativen Analyse oder Erfassung des Schmiermittels und folglich der Erzeugung eines Mengensignals dient, ist eine Detektionseinrichtung, die auf kapazitiver Grundlage arbeitet und die eine oder mehrere kapazitiv arbeitende Sensoren aufweist, die jeweils zumindest eine in einem Abstand zum Band angeordnete Fühlerplatte aufweisen, die mit dem Band jeweils einen Kondensator bilden, dessen Kapazität von der auf dem Band angeordneten Menge des Materials, insbesondere Schmiermittels, abhängt.

Dabei geht die Erfindung von der Erkenntnis aus, dass sich die auf der Bandoberfläche befindliche Schmiermittelmenge besonders einfach und zuverlässig mit kapazitiven Mitteln überwachen bzw. bestimmen lässt, und zwar durch Einbeziehung des aus einem elektrisch leitfähigen Material hergestellten Bandes, z. B. Stahlbandes. Es werden eine oder mehrere Fühlerplatten in einem vorgegebenen festen Abstand zu dem Band angeordnet, so dass die Fühlerplatte mit dem Band einen Kondensator bildet, dessen Kapazität empfindlich durch die dielektrische Wirkung des Schmiermittels, z. B. Öls, beeinflusst wird. Denn die Permitivität bzw. relative Dielektrizitätskonstante Er des Schmiermittels, z. B. Öls weicht mit einem Wert von etwa 2 sehr deutlich von der der Luft mit einem Wert von etwa 1 ab. Da diese Permitivität aufgrund des Zusammenhangs C=ε × εᵣx Ald linear in die Kapazität des von Fühlerplatte und Band gebildeten Plattenkondensators eingeht, ist die Kapazität des auf diese Weise mit dem Band (z. B. Pressband einer kontinuierlichen Presse) gebildeten Kondensators ein empfindliches und zuverlässiges Maß für die auf dem Band und folglich innerhalb des Plattenkondensators angeordnete Schmiermittelmenge. Dabei kann ein grundsätzlich bekanntes Band bzw. Pressband aus elektrisch leitfähigem Material, z. B. ein Stahlband verwendet werden. Es ist jedoch ebenso möglich, ein Band, z. B. Pressband zu verwenden, das lediglich eine elektrisch leitfähige Oberfläche aufweist, die mit dem Schmiermittel benetzt ist.

Kapazitive Sensoren, die auf Basis der Veränderung der elektrischen Kapazität eines Kondensators arbeiten, sind zwar aus dem Stand der Technik bekannt, z. B. als Drucksensoren, Abstandssensoren, Näherungsschalter, Spaltsensoren, Wegsensoren oder auch als Feuchtigkeitssensoren. Für den Einsatz in kontinuierlichen Pressen und insbesondere für die quantitative Überwachung des Schmierzustandes von mit Schmiermittel beaufschlagten umlaufenden Bändern ist eine solche Technologie jedoch bislang nicht in Betracht gezogen worden.

In besonders vorteilhafter Weise besteht bei der ersten Detektionseinrichtung die Möglichkeit, den Schmierzustand des Bandes selektiv bzw. individuell über die Bandbreite zu analysieren bzw. zu überwachen, so dass nicht nur ein über die Breite gemittelter Schmierzustand erfasst werden kann, sondern insbesondere auch eine über die Breite ungleichmäßige Verteilung erfasst wird. Dieses gelingt bei der ersten Detektionseinrichtung z. B. mit den kapazitiv arbeitenden Sensoren. Denn in bevorzugter Weiterbildung wird vorgeschlagen, dass über die Breite des Bandes nebeneinander mehrere Fühlerplatten angeordnet sind, die mit dem Band nebeneinander mehrere Kondensatoren bilden, die jeweils unterschiedlichen Breitenpositionen des Bandes zugeordnet sind. Dabei besteht z. B. die Möglichkeit, über die Breite des Bandes zumindest fünf Fühlerplatten und folglich fünf Kondensatoren zu realisieren, so dass eine Überwachung des Schmierzustandes in fünf nebeneinander angeordneten Spuren möglich ist. Durch Aufteilung und Anzahl der Spuren kann eine Anpassung an die jeweiligen Gegebenheiten erfolgen, und zwar derart, dass durch eine höhere Anzahl von Spuren eine feinere bzw. selektivere Überwachung des Bandes über die Bandbreite ermöglicht wird.

Interessant ist die Tatsache, dass das Band, z. B. das Pressband einer kontinuierlichen Presse in der Regel ohnehin aus einem elektrisch leitfähigen Material gefertigt ist (z. B. als Stahlband) und dass ein solches Band üblicherweise ohnehin geerdet ist, so dass auf einfache Weise mit den Fühlerplatten ein oder mehrere Kondensatoren für eine kapazitive Schmiermittelüberwachung realisiert werden können.

In bevorzugter Weiterbildung einer z. B. als kapazitiv arbeitend ausgebildeten ersten Detektionseinrichtung ist vorgesehen, dass der oder die Sensoren (jeweils) eine (elektronische) Schaltungsanordnung aufweisen, die mit der jeweiligen Fühlerplatte verbunden ist oder in welche die Fühlerplatte integriert ist und mit der das von der Schmiermittelmenge abhängige Messsignal erzeugbar ist. Bestandteil des Sensors ist folglich nicht nur der Kondensator selbst, sondern eine elektronische Schaltungsanordnung, bei der jedoch auf bekannte Maßnahmen zur Verschaltung von Kondensatoren bzw. Kondensatorplatten im Zuge einer kapazitiven Messung zurückgegriffen werden kann. So kann es sich z. B. bei der Schaltungsanordnung um einen elektronischen Schwingkreis und folglich einen LC-Oszillator handeln, der einerseits von zumindest dem Kondensator (aus Fühlerplatte und Band) und andererseits einer zusätzlichen Spule gebildet wird, wobei die Frequenz des Schwingkreises bzw. des Oszillators als Messsignal von der Kapazität des Kondensators und damit von der innerhalb des Kondensators angeordneten Schmiermittelmenge abhängt. Die durch die elektrische Wirkung des Öls erzeugte Veränderung der Kapazität kann folglich in Form einer Veränderung der Frequenz eines LC-Oszillators erfasst werden, wobei sich die Frequenz des Oszillators verändert, wenn die Ölmenge zwischen Platte und Band kleiner oder größer wird, z. B. kleiner oder größer als die gewünschte Ölmenge wird. Der erfindungsgemäße schmiermittelabhängige bzw. ölabhängige Kondensator wird folglich als frequenzbeeinflussendes Element in einem LC-Oszillator und folglich als frequenzbestimmendes Bauteil eingesetzt. Sofern sich die Ölmenge ändert, ändert sich auch die Frequenz.

Die erste Detektionseinrichtung, die auf kapazitiver Basis arbeitet, kann für die Sensoren mit einer oder mit mehreren (einzelnen) Auswerteeinrichtungen ausgerüstet sein, mit denen durch Auswertung des Messsignals die Schaltungsanordnung ein von dem Messsignal abhängiges und den Schmierzustand repräsentierendes Informationssignal und folglich das Mengensignal der ersten Detektionseinrichtung erzeugbar ist. Dazu kann z. B. in der oder den Auswerteeinrichtungen das Messsignal mit einem oder mit mehreren Vergleichswerten oder mit einem oder mehreren Vergleichsintervallen verglichen werden, wobei durch Vergleich des Messsignals mit der oder den Vergleichswerten bzw. Vergleichsintervallen das Mengensignal erzeugbar ist, z. B. bei Überschreiten oder Unterschreiten eines Vergleichswertes oder eines Vergleichsintervalls. Eine solche Auswerteeinrichtung lässt sich z. B. schaltungstechnisch sehr einfach durch eine grundsätzlich bekannte Filterschaltung realisieren, die bei entsprechender Abweichung ein von der Menge abhängiges Mengensignal erzeugt, das z. B. an eine Meldeeinrichtung weitergeleitet werden kann.

Alternativ zu der beschriebenen Schaltungsanordnung in Form eines LC-Oszillators können auch andere grundsätzlich bekannte Schaltungsanordnungen, z. B. PLL-Schaltungen ("phase locked loop") zum Einsatz kommen.

Insgesamt steht im Vordergrund der beschriebenen kapazitiven, ersten Detektionseinrichtung die Überwachung der Schmiermittelmenge, und zwar bevorzugt breitenselektiv über die Breite des Bandes. Da dieses Messprinzip darauf beruht, dass die Permitivität eines Schmiermittels, z. B. Öls, sehr deutlich von der Permitivität von Luft abweicht, ist zu beobachten, dass sich das Schmiermittel nicht ohne Weiteres von Stoffen mit gleicher oder sehr ähnlicher Permitivität unterscheiden lässt. In einer Presse kommt insbesondere eine Verschmutzung des Schmiermittels mit Holzpartikeln in Betracht und solche Holzpartikel haben eine sehr ähnliche Permitivität wie die üblicherweise eingesetzten flüssigen Schmiermittel, so dass mit einer z. B. kapazitiv arbeitenden ersten Detektionseinrichtung keine Unterscheidung zwischen Schmiermittel und Holz oder auch von mit Holzpartikeln verunreinigten Schmiermitteln erfolgen kann. Aus diesem Grund wird die erste Detektionseinrichtung erfindungsgemäß mit der zweiten Detektionseinrichtung kombiniert.

Die zweite Detektionseinrichtung kann z. B. eine bildgebende Einrichtung aufweisen bzw. als bildgebende Einrichtung ausgebildet sein. Bei einer solchen bildgebenden Einrichtung kann es sich z. B. um eine Kamera handeln, mit der Bilder des Materials auf dem Band aufnehmbar sind. Ferner kann diese zweite Detektionseinrichtung eine Auswerteeinheit, z. B. Rechnereinheit aufweisen, mit der die aufgenommenen Bilder derart auswertbar sind, dass die Art des Materials ermittelbar und gegebenenfalls das Materialsignal erzeugbar ist. Dabei geht die Erfindung von der Erkenntnis aus, dass eine optische Überwachung der Bandoberfläche zwar in der Regel nicht für eine quantitative Mengenauswertung, jedoch gut für eine materialsensitive Auswertung geeignet ist. Denn durch eine geeignete Analyse lässt sich anhand der aufgenommenen Bilder und insbesondere durch Vergleich mit abgespeicherten Bildern, Bilddaten, Mustern oder dergleichen auf Basis geeigneter Algorithmen zwischen einem sauberen Schmiermittel einerseits und einem übermäßig verschmutzten Schmiermittel andererseits oder auch Holzpartikeln, die in einem geschmierten oder auch nicht geschmierten Bereich auf dem Band sein können, unterscheiden. Damit können die im Zusammenhang mit der ersten Detektionseinrichtung gegebenen Nachteile hinsichtlich der fehlenden Materialsensitivität mit der zweiten Detektionseinrichtung kompensiert werden. Die zweite Detektionseinrichtung kann optional eine zusätzliche Lichtquelle aufweisen, mit der die zu analysierende Oberfläche des Bandes gleichmäßig ausgeleuchtet wird. Auf diese Weise können gleichbleibende, standardisierte Lichtverhältnisse für die Aufnahme der Bilder mit der Kamera geschaffen werden. Dieses erhöht die Qualität der Aufnahme und insbesondere die Qualität des Vergleichs mit den hinterlegten Vergleichsdaten bzw. Bildern.

Alternativ können für die zweite Detektionseinrichtung andere Einrichtungen bzw. Analyseverfahren eingesetzt werden.

So kann eine Detektionseinrichtung, z. B. als Fluoreszenzmessvorrichtung ausgebildet sein, die zumindest eine elektromagnetische Strahlungsquelle aufweist, mit der elektromagnetische Strahlung eines ersten Wellenlängenbereichs erzeugbar ist und die zumindest einen Sensor aufweist, wobei mit dem Sensor elektromagnetische Strahlung eines zweiten Wellenlängenbereichs, insbesondere Fluoreszenzstrahlung, detektierbar ist, der von dem ersten Wellenlängenbereich abweicht, vorzugsweise oberhalb des ersten Wellenlängenbereichs liegt. So lässt sich z. B. die zu analysierende Oberfläche mit Wellenlänge in einem Wellenlängenbereich von 190 bis 420 nm, insbesondere im Bereich von 250 nm bis 420 nm und besonders bevorzugt von 300 nm bis 365 nm bestrahlen. Diese Bestrahlung führt aufgrund der Schmiermittelbestandteile bzw. spezieller Schmiermitteladditive zur Anregung der atomaren bzw. molekularen Zustände, gefolgt von spontaner Emission von elektromagnetischer Strahlung, die energieärmer ist und folglich größere Wellenlängen als die zuvor absorbierte Strahlung aufweist. Insofern wird der Sensor dieser Fluoreszenzmessvorrichtung bevorzugt so eingerichtet, dass die Strahlung in dem gewünschten Wellenlängenbereich zuverlässig und effektiv detektiert wird, z. B. in einem Wellenlängenbereich von 420 nm bis 575 nm, insbesondere 420 nm bis 520 nm, besonders bevorzugt 440 nm bis 495 nm.

Eine Fluoreszenzmessvorrichtung des beschriebenen Typs kann als zweite Detektionseinrichtung eingesetzt werden, die für eine Unterscheidung der auf dem Band befindlichen Materialien eingesetzt wird. Denn durch Analyse der emittierten Fluoreszenzstrahlung können bei geeigneter Ausgestaltung der Sensoren Rückschlüsse auf die Art des Materials erfolgen, und zwar insbesondere dann, wenn dem Schmiermittel spezielle Additive beigegeben sind oder beigegeben werden, die in der emittierten Fluoreszenzstrahlung eine Unterscheidung des Öls von z. B. Holzpartikeln oder anderen Schmutzpartikeln ermöglichen. In diesem Zusammenhang besteht z. B. die Möglichkeit, mit mehreren Sensoren die emittierte Fluoreszenzstrahlung in unterschiedlichen Frequenzbereichen bzw. Wellenlängenbereichen aufzunehmen, um Unterschiede zwischen verschiedenen Materialien zu ermitteln.

Es kann eine Detektionseinrichtung in einer weiteren Ausführungsform auch als Interferenzmessvorrichtung ausgebildet sein, die zumindest eine elektromagnetische Strahlungsquelle, z. B. eine Röntgenquelle aufweist und die zumindest einen Sensor aufweist, z. B. eine CCD-Kamera. Mit diesem Sensor lässt sich z. B. ein Interferenzmuster aufnehmen, das aufgrund der am Band reflektierten Strahlung und der durch das auf dem Band befindliche Material gebeugten Strahlung entsteht. Optional liegt es im Rahmen der Erfindung, eine solche Interferenzmessvorrichtung als zweite Detektionseinrichtung für eine materialsensitive Auswertung einzusetzen.

Die Erfindung betrifft im Übrigen nicht nur eine Vorrichtung zur Überwachung des Schmierzustandes, sondern auch ein entsprechendes Verfahren zur Überwachung eines mit einem Schmiermittel beaufschlagten, umlaufenden Bandes für den Transport von Pressgut, insbesondere in einer kontinuierlich arbeitenden Presse, wobei mit zumindest einer im Bereich des Bandes angeordneten ersten Detektionseinrichtung eines ersten Typs und mit zumindest einer im Bereich des Bandes angeordneten zweiten Detektionseinrichtung eines zweiten Typs zumindest ein Signal erzeugt wird. Dieses Verfahren ist durch die Merkmale des Anspruchs 9 definiert. Es ist vorgesehen, dass mit der ersten Detektionseinrichtung ein von der Menge des auf dem Band befindlichen Materials abhängiges Mengensignal erzeugt wird und dass mit der zweiten Detektionseinrichtung ein von der Art des auf dem Band befindlichen Materials abhängiges Materialsignal erzeugt wird und dass aus dem Materialsignal zwischen einem auf dem Band befindlichen sauberen Schmiermittel einerseits und auf dem Band befindlichen Holzpartikeln oder einem mit Holzpartikeln verunreinigten Schmiermittel andererseits unterschieden wird.

Ein solches Verfahren kann in vorteilhafter Weise unter Verwendung einer erfindungsgemäßen Vorrichtung durchgeführt werden.

Gegenstand der Erfindung ist außerdem eine kontinuierlich arbeitende Presse, die zumindest ein oberes endlos umlaufendes Band (Pressband) und ein unteres endlos umlaufendes Band (Pressband) aufweist, wobei das Pressgut durch den Pressspalt zwischen dem oberen und dem unteren Band hindurchgeführt wird. Bevorzugt handelt es sich um eine Pressvorrichtung, bei der das Pressgut unter Anwendung von Druck und/oder Wärme zu einer Platte bzw. einem endlosen Plattenstrang verpresst wird. Alternativ kann es sich bei einer solchen Presse auch um eine Vorwärmeinrichtung handeln, die z. B. in einem kontinuierlichen Pressprozess der Vorwärmung des Pressgutes dient, bevor dieses in eine kontinuierlich arbeitende Presse zur Herstellung der eigentlichen Platte einläuft. Bevorzugt werden solche kontinuierlich arbeitenden Pressen nach Art einer Doppelbandpresse erfasst, bei denen die Pressbänder unter Zwischenschaltung von Wälzkörpern, z. B. von Rollstangen oder Rollstäben, an der jeweiligen Pressenplatte abgestützt sind. Die Erfindung betrifft jedoch alternativ auch Pressen mit umlaufenden Pressbändern anderer Bauart, z. B. Kalanderpressen. Stets ist eine solche Presse mit einer Vorrichtung zur Überwachung des Schmierzustandes der beschriebenen Art ausgerüstet, d.h., die Erläuterungen zur Schmierung und Überwachung des Bandes betreffen bei einer Presse bevorzugt das jeweilige obere Pressband und/oder das jeweilige untere Pressband. Gegenstand einer solchen Presse ist in der Regel auch eine Auftragsvorrichtung, mit der ein Schmiermittel auf das obere und/oder das untere Band (unmittelbar oder mittelbar) aufbringbar ist. Die erfindungsgemäße Presse ist folglich dadurch gekennzeichnet, dass die Presse mit einer Vorrichtung zur Überwachung des Schmierzustandes der beschriebenen Art ausgerüstet ist.

In entsprechender Weise betrifft die Erfindung schließlich auch ein Verfahren zum Betrieb einer derartigen Presse, das dadurch gekennzeichnet ist, dass der Schmierzustand der Presse mit einem erfindungsgemäß ausgestalteten Verfahren zur Überwachung eines mit einem Schmiermittel beaufschlagten, umlaufenden Bandes für den Transport von Pressgut überwacht wird.

Im Folgenden wird die Erfindung anhand einer lediglich ein Ausführungsbeispiel darstellenden Zeichnung näher erläutert. Es zeigen
- Fig. 1: schematisch vereinfacht eine kontinuierlich arbeitende Presse mit einer Vorrichtung zur Überwachung des Schmierzustandes der Pressbänder,
- Fig. 2: schematisch vereinfacht eine Vorrichtung zur Überwachung des Schmierzustandes innerhalb einer Presse nach Fig. 1 in einer erfindungsgemäßen ersten Ausführungsform,
- Fig. 3: eine Schaltungsanordnung der ersten Detektionseinrichtung einer Vorrichtung nach Fig. 2,
- Fig. 4: schematisch vereinfacht eine Vorrichtung nach Fig. 2 in einer abgewandelten und nicht beanspruchten Ausführungsform, und
- Fig. 5: eine weitere Ausführungsform der Erfindung.

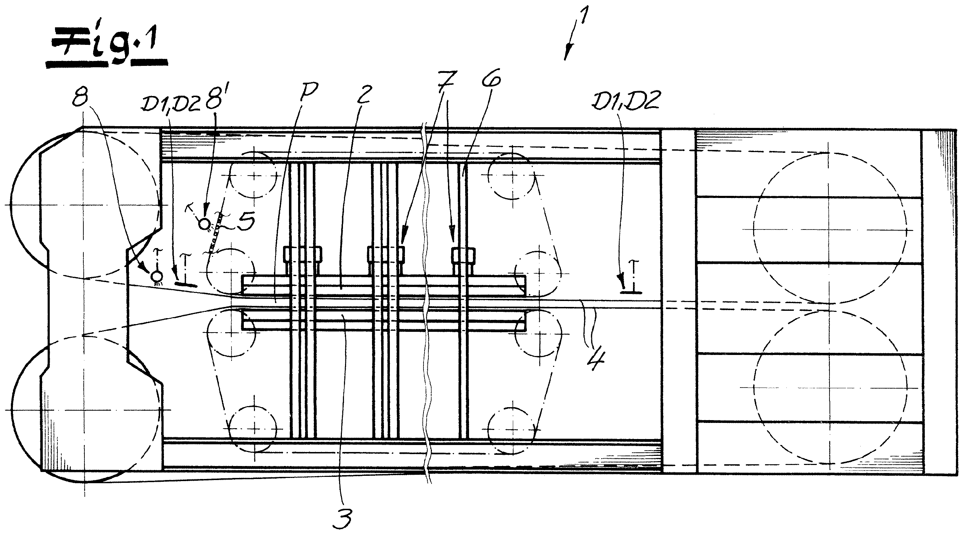

In Fig. 1 ist eine kontinuierlich arbeitende Presse 1 dargestellt, die z. B. zum Verpressen von Pressgutmatten zu Pressgutplatten im Zuge der Herstellung von Spanplatten, Faserplatten oder anderen Holzwerkstoffplatten geeignet und bestimmt ist. Die kontinuierliche Presse 1 weist in ihrem grundsätzlichen Aufbau ein Pressenoberteil mit einer oberen beheizbaren Pressenplatte 2 und ein Pressenunterteil mit einer unteren beheizbaren Pressenplatte 3 auf. Die beheizbaren Pressenplatten 2, 3 werden auch als Heizplatten bezeichnet. Im Pressenoberteil sowie im Pressenunterteil sind endlos umlaufende Bänder 4 geführt, die als Pressbänder 4 bezeichnet werden und z. B. aus Stahl gefertigt sind. Zwischen den Pressbändern 4 wird ein Pressspalt P gebildet, durch den das Pressgut hindurchgeführt wird. Die Pressbänder 4 sind unter Zwischenschaltung von Rollstäben bzw. Rollstangen 5 gegen die Pressenplatten 2, 3 abgestützt. Die Rollstäbe 5 sind dabei z. B. an Rollstabketten geführt, die nicht im Detail dargestellt sind. Das Pressgut wird innerhalb der Presse unter Anwendung von Druck und Wärme zu einem aus der Presse auslaufenden Plattenstrang verpresst. Die dazu erforderlichen Presskräfte werden mit Kraftmitteln, insbesondere mit hydraulischen Presszylindern 7 aufgebracht, mit denen z. B. die obere Pressenplatte 2 oder die untere Pressenplatte 3 beaufschlagt wird. Diese Presszylinder 7 sind am Pressengestell, z. B. an dessen Pressenrahmen 6 abgestützt. Die Fig. 1 zeigt dabei lediglich beispielhaft eine Oberkolbenpresse, bei der die obere Pressenplatte 2 mit den Presszylindern 7 beaufschlagt wird.

Die Pressbänder werden mit einem Schmiermittel beaufschlagt, insbesondere mit einem flüssigen Schmiermittel, z. B. mit Öl. Dabei ist in Fig. 1 die Möglichkeit angedeutet, das jeweilige Pressband unmittelbar mit dem Schmiermittel zu beaufschlagen, z. B. durch eine geeignete Auftragsvorrichtung 8, die z. B. als sich quer zur Bandlaufrichtung über die Bandbreite erstreckende Bedüsungsvorrichtung ausgebildet sein kann. Alternativ oder ergänzend kann jedoch eine in Fig. 1 ebenfalls angedeutete Auftragsvorrichtung 8' vorgesehen sein, die die umlaufenden Rollstangen 5 beaufschlagt, so dass über die Rollstangen eine mittelbare Beaufschlagung des Pressbandes mit dem Schmiermittel erfolgt.

Dabei zeigt Fig. 1 lediglich die Schmierung des oberen Bandes. Selbstverständlich sind entsprechende Maßnahmen auch im Bereich des unteren Bandes vorgesehen. Stets erfolgt die Beaufschlagung der dem Pressgut abgewandten "inneren" Oberfläche des jeweiligen Pressbandes 4.

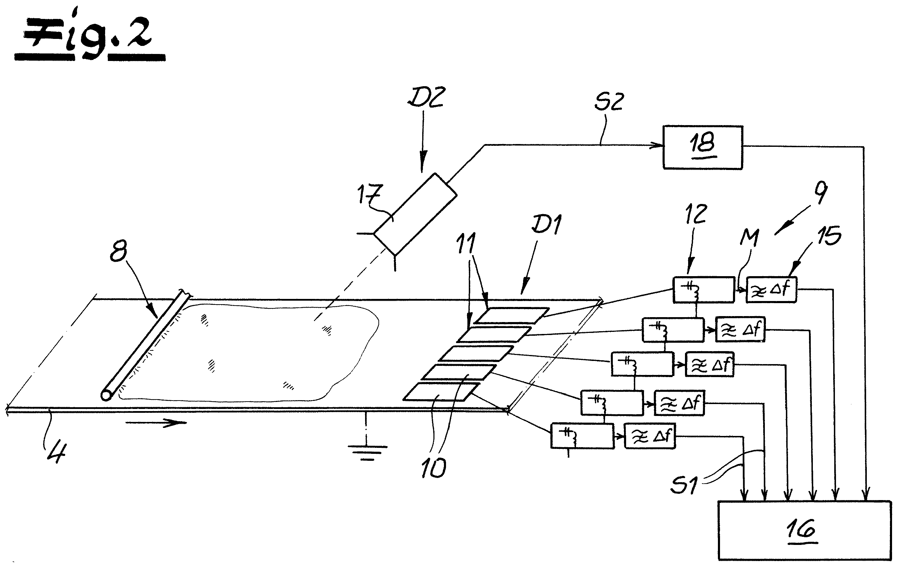

Die Presse 1 ist mit einer Vorrichtung zur Überwachung des Schmierzustandes der mit dem Schmiermittel beaufschlagten, umlaufenden Bänder ausgerüstet. Diese Vorrichtung ist beispielhaft in Fig. 2 dargestellt. Sie weist einerseits eine erste Detektionseinrichtung D1 und andererseits eine zweite Detektionseinrichtung D2 auf. Die erste Detektionseinrichtung D1 ist dazu eingerichtet, ein von der Menge des auf dem Band 4 befindlichen Materials abhängiges Mengensignal S1 zu erzeugen. Zur Unterscheidung eines auf dem Band 4 befindlichen Schmiermittels von auf dem Band 4 befindlichen Holzpartikeln oder von einem mit Holzpartikeln verunreinigten Schmiermittel ist die zweite Detektionseinrichtung D2 dazu eingerichtet, ein von der Art des auf dem Band 4 befindlichen Materials abhängiges Materialsignal S2 zu erzeugen. Durch Kombination zweier unterschiedlicher Messeinrichtungen bzw. zweier unterschiedlicher Messverfahren lässt sich folglich nicht nur zuverlässig die Schmiermittelmenge auf dem Band überwachen, sondern es kann auch eine Unterscheidung zwischen unterschiedlichen Materialien erfolgen. Damit kann z. B. zuverlässig vermieden werden, dass bei einer quantitativen Überwachung des Schmiermittels bezogen auf die Menge des Schmiermittels fehlerhafte Rückschlüsse gezogen werden, wenn sich die gemessene Menge nicht auf ein Schmiermittel, sondern auf Holzpartikel bezieht. Insofern kann die erste Detektionseinrichtung D1 gezielt auf eine mengenmäßige Erfassung ausgelegt sein, ohne dass eine Unterscheidung zwischen z. B. einem Schmiermittel und Holzpartikeln erfolgen muss. In dem Ausführungsbeispiel nach Fig. 2 ist die erste Detektionseinrichtung D1 als kapazitiv arbeitende Vorrichtung ausgebildet. Sie weist mehrere im Bereich des Bandes angeordnete und über die Bandbreite verteilte Sensoren 9 auf. Diese Sensoren 9 sind als kapazitiv arbeitende Sensoren ausgebildet, die jeweils zumindest eine in einem Abstand d zum Band 4 angeordnete Fühlerplatte 10 aufweisen, die mit dem Band 4 jeweils einen Kondensator 11 bilden, dessen Kapazität von der auf dem Band angeordneten Schmiermittelmenge abhängt. Dabei ist in Fig. 2 erkennbar, dass über die Breite des Bandes 4 nebeneinander mehrere Fühlerplatten 10 angeordnet sind, die mit dem Band gemeinsam mehrere nebeneinander angeordnete Kondensatoren 11 bilden, die unterschiedlichen Breitenpositionen des Bandes zugeordnet sind. Im Ausführungsbeispiel sind fünf nebeneinander angeordnete Fühlerplatten 10 und folglich fünf nebeneinander angeordnete Sensoren dargestellt, so dass eine Überwachung des Schmierzustandes in fünf einzelnen Spuren auf dem Band möglich ist.

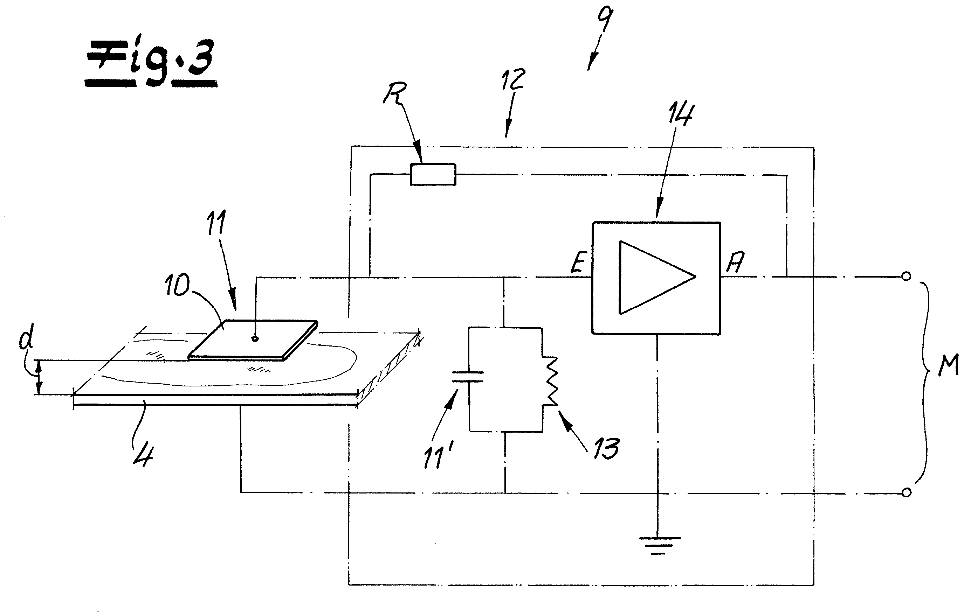

Die Sensoren 9 weisen jeweils eine Schaltungsanordnung 12 auf, die mit der jeweiligen Fühlerplatte 10 verbunden ist bzw. in welche die jeweilige Fühlerplatte 10 integriert ist (vgl. z. B. Fig. 3). Mit dieser Schaltungsanordnung 12 kann (für die jeweilige Breitenposition) ein von der Schmiermittelmenge abhängiges Messsignal M erzeugt werden. In dem dargestellten Ausführungsbeispiel ist diese Schaltungsanordnung 12 als Schwingkreis und folglich als LC-Oszillator ausgebildet, welche einerseits den Kondensator 11 und andererseits eine Spule 13 aufweisen. In Fig. 3 ist dabei eine Variante angedeutet, bei der zunächst ein Basisschwingkreis mit der Spule 13 und einem unmittelbar zugeordneten Kondensator 11' vorgesehen ist, dem der Kondensator 11 aus Fühlerplatte 10 und Band 4 parallel geschaltet ist, so dass sich die Kapazitäten aus 11 und 11' addieren. Ferner sind ein Verstärker 14 mit Eingang E und Ausgang A sowie ein Widerstand R dargestellt. Der Ausgang dieser Schaltungsanordnung 12 gemäß Fig. 3 liefert ein Frequenzsignal als Messsignal, d.h., die Frequenz des Schwingkreises hängt von der Kapazität des Kondensators 11 und damit von der innerhalb des Kondensators 11 auf dem Band 4 angeordneten Schmiermittelmenge ab. Die Frequenz des Oszillators verändert sich folglich, wenn die Ölmenge zwischen Platte und Band kleiner oder größer wird. Der schmiermittelabhängige Kondensator 11 wird folglich in einem LC-Oszillator als frequenzbestimmendes Bauteil eingesetzt, so dass die Frequenz als Messsignal M zur Verfügung steht.

Die Sensoren 9 (bzw. deren Schaltungen 12) sind mit einer oder mit mehreren Auswerteeinrichtungen 15 verbunden, die im Ausführungsbeispiel als Filterschaltungen 15 ausgebildet sind. Sie sind in den Figuren schematisch vereinfacht als getrennte Komponenten dargestellt, können jedoch selbstverständlich auch schaltungstechnisch untereinander und/oder mit den Schaltungen 12 kombiniert sein. Mit den Auswerteeinrichtungen 15 werden durch Auswertung des Messsignals jeweils die von dem Messsignal M abhängigen und den Schmierzustand repräsentierenden Mengensignal S1 erzeugt. Dazu können die Messsignale M in den Auswerteeinrichtungen 15 jeweils mit einem oder mit mehreren Vergleichswerten oder mit einem Vergleichsintervall verglichen werden und in Abhängigkeit von dem Ergebnis des Vergleichs kann das Informationssignal S1 erzeugt werden, z. B. bei Überschreiten oder bei Unterschreiten eines Vergleichswertes oder eines Vergleichsintervalls. So besteht z. B. die Möglichkeit, dass ein Informationssignal S1 erzeugt und an eine Meldeeinrichtung 16 übermittelt wird, wenn für die jeweilige Breitenposition eine Unterschmierung oder eine Überschmierung festgestellt wird. Schaltungstechnisch lässt sich dieses z. B. durch eine Filterschaltung realisieren, d.h., das Informationssignal wird erzeugt, wenn die gemessene Frequenz von einer vorgegebenen Frequenz abweicht oder aus einem vorgegebenen Frequenzbereich fällt.

Insgesamt ist die erste Detektionseinrichtung gemäß Fig. 2 auf die mengenmäßige Überwachung des Schmiermittels ausgelegt. Beispielsweise mit dem beschriebenen kapazitiven Prinzip lässt sich jedoch gegebenenfalls nicht erkennen, ob sich die innerhalb des Kondensators angeordnete Materialmenge tatsächlich auf ein (sauberes) Schmiermittel bezieht oder auf ein mit Holzpartikeln verschmutztes Schmiermittel oder gar auf eine Schicht aus Holzpartikeln. Denn Holzpartikel einerseits und das Schmiermittel andererseits beeinflussen die Permitivität des Kondensators in etwa in gleicher Weise.

Aus diesem Grund ist erfindungsgemäß die in Fig. 2 ebenfalls dargestellte zweite Detektionseinrichtung D2 vorgesehen, die hier eine bildgebende Einrichtung, nämlich eine Kamera 17 sowie eine Rechnereinheit 18 aufweist. Mit der Kamera können ein oder mehrere Bilder des Materials auf dem Band 4 aufgenommen werden, die anschließend mit der Rechnereinheit 18 derart ausgewertet werden, dass die Art des Materials ermittelt wird bzw. dass ein Materialsignal S2 erzeugt wird, das eine Information darüber enthält, ob es sich bei dem Material um ein Schmiermittel in der gewünschten Zusammensetzung handelt oder um ein übermäßig mit Holzpartikeln verschmutztes Schmiermittel oder gar um Holzpartikel. Dabei kann die Anzahl der Bilder pro Zeiteinheit der Geschwindigkeit des Pressbandes im Betrieb angepasst sein. Das erzeugte Materialsignal S2 wird ebenfalls der Melde- und Rechnereinheit 16 zugeführt, die auch die Mengensignale S1 erhält (vgl. Fig. 2). Optional kann die materialsensitive Analyse mit der zweiten Detektionseinrichtung D2 und für verschiedene Breitenbereiche bzw. Spuren erfolgen, die den von der ersten Detektionseinrichtung D1 erzeugten Spuren entsprechen. Diese Option ist nicht dargestellt.

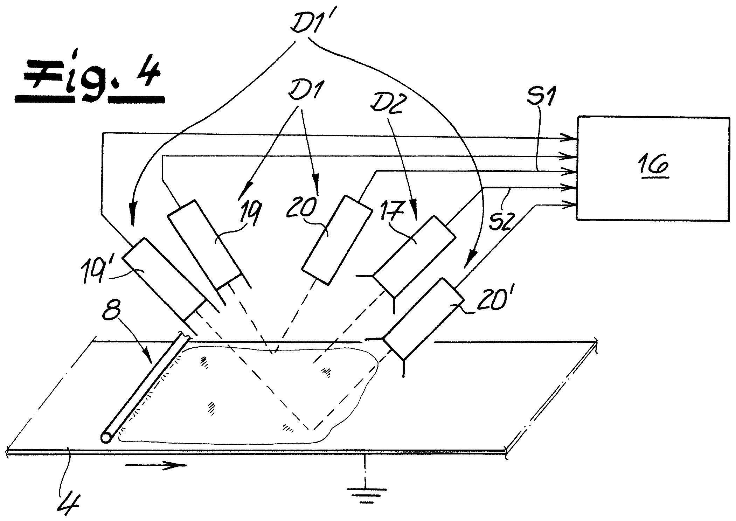

Fig. 4 zeigt eine Ausführungsform, die nicht der beanspruchten Erfindung entspricht, bei der die erste Detektionseinrichtung D1 für die Erzeugung des Mengensignals S1 als Fluoreszenzmessvorrichtung ausgebildet ist. Diese weist eine elektromagnetische Strahlungsquelle 19, z. B. einen Laser, auf, der elektromagnetische Strahlung in einem ersten Wellenlängenbereich erzeugt und damit die Oberfläche des Bandes bestrahlt. Ferner ist ein Sensor 20 vorgesehen, der Fluoreszenzstrahlung ermittelt, die aus der auf dem Band befindlichen Schmiermittelschicht emittiert wird und die eine größere Wellenlänge und folglich geringere Frequenz aufweist als die eingestrahlte Laserstrahlung. Mit Hilfe einer solchen Fluoreszenzmessung lässt sich durch geeignete Auswertung mit dem Rechnersystem 16 ebenfalls auf die Schmiermittelmenge schließen. Diese erste Detektionseinrichtung D1 wird wiederum mit der Detektionseinrichtung D2 kombiniert, die bereits im Zusammenhang mit Fig. 2 erläutert wurde.

In einer nicht beanspruchten Ausführungsform kann optional (oder ergänzend) zu der ersten Detektionseinrichtung D1 als Fluoreszenzmessvorrichtung die erste Detektionseinrichtung D1 auch als Interferenzmessvorrichtung ausgebildet sein. Auch eine solche ist in Fig. 4 dargestellt. Es sind einerseits eine Röntgenquelle 19' und andererseits ein Sensor 20' erkennbar, die wiederum mit dem Rechnersystem verbunden sind. Mit dem Sensor 20`, der z. B. als CCD-Kamera ausgebildet sein kann, kann ein Interferenzmuster aufgenommen werden, das ebenfalls Informationen über die Schmiermittelmenge enthält und insofern mit der ersten Detektionseinrichtung D1 kombinierbar ist.

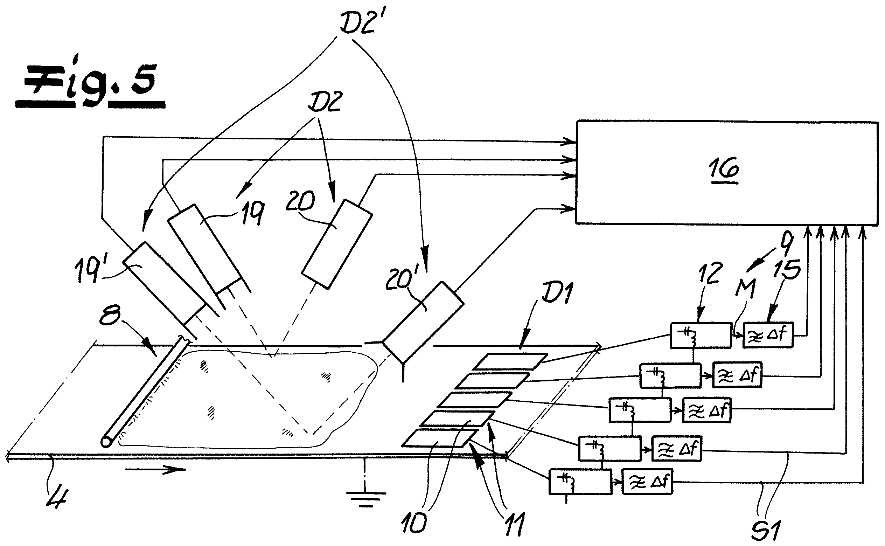

In Fig. 5 ist eine weitere Ausführungsform der Erfindung dargestellt, bei der die erste Detektionseinrichtung D1 wiederum als kapazitive Einrichtung ausgebildet ist, die bereits im Zusammenhang mit Fig. 2 erläutert wurde. In diesem Fall kann die zweite Detektionseinrichtung, die für die Materialanalyse verantwortlich ist, entweder als Fluoreszenzmessvorrichtung D2 oder als Interferenzmessvorrichtung D2' ausgebildet sein.

## Patentansprüche

1. Vorrichtung zur Überwachung des Schmierzustandes eines mit einem Schmiermittel beaufschlagten, umlaufenden Bandes (4) für den Transport von Pressgut, insbesondere in einer kontinuierlich arbeitenden Presse (1),
mit zumindest einer, im Gebrauch, im Bereich des Bandes (4) angeordneten ersten Detektionseinrichtung (D1) eines ersten Typs und mit zumindest einer, im Gebrauch, im Bereich des Bandes (4) angeordneten zweiten Detektionseinrichtung (D2) eines zweiten Typs,
wobei die erste Detektionseinrichtung (D1) dazu eingerichtet ist, ein von der Menge des auf dem Band (4) befindlichen Materials abhängiges Mengensignal (S1) zu erzeugen,
wobei zur Unterscheidung eines auf dem Band (4) befindlichen Schmiermittels von auf dem Band (4) befindlichen Holzpartikeln oder von einem mit Holzpartikeln verunreinigten Schmiermittel die zweite Detektionseinrichtung (D2) dazu eingerichtet ist, ein von der Art des auf dem Band (4) befindlichen Materials abhängiges Materialsignal (S2) zu erzeugen, welches davon abhängt, ob sich auf dem Band ein sauberes Schmiermittel oder ein mit Holzpartikeln verschmutztes Schmiermittel oder Holzpartikel befinden,
**dadurch gekennzeichnet, dass** die erste Detektionseinrichtung (D1) einen oder mehrere kapazitiv arbeitende Sensoren (9) aufweist, die jeweils zumindest eine, im Gebrauch,
in einem Abstand zum Band (4) angeordnete Fühlerplatte (10) aufweisen, die mit dem Band jeweils einen Kondensator (11) bildet, dessen Kapazität von der auf dem Band (4) angeordneten Menge des Materials, nämlich von sauberem Schmiermittel, Holzpartikeln oder mit Holzpartikeln verschmutztem Schmiermittel, abhängt.

2. Vorrichtung nach Anspruch 1, **gekennzeichnet durch** ein Melde- und/oder Rechnersystem (16), mit dem das Mengensignal (S1) und das Materialsignal (S2) anzeigbar sind und/oder mit dem das Mengensignal (S1) und das Materialsignal (S2) derart auswertbar sind, dass aus dem Materialsignal (S2) die Art des auf dem Band befindlichen Materials derart ermittelbar ist, dass zwischen einem auf dem Band (4) befindlichen sauberen Schmiermittel einerseits und auf dem Band befindlichen Holzpartikeln oder einem mit Holzpartikeln verunreinigten Schmiermittel andererseits unterschieden wird.

3. Vorrichtung nach Anspruch 1 oder 2, **dadurch gekennzeichnet, dass** die zweite Detektionseinrichtung (D2) eine Kamera (17) und eine Rechnereinheit (18) aufweist, wobei mit der Kamera ein oder mehrere Bilder des Materials auf dem Band aufnehmbar sind, welche mit der Rechnereinheit (18) derart auswertbar sind, dass die Art des Materials ermittelbar und gegebenenfalls das Materialsignal (S2) erzeugbar ist.

4. Vorrichtung nach Anspruch 3, **dadurch gekennzeichnet, dass** in der Rechnereinheit (18) der zweiten Detektionseinrichtung (D2) Informationen, z. B. Bilddaten, Muster oder dergleichen verschiedener Materialien, insbesondere des sauberen Schmiermittels einerseits und von Holzpartikeln und/oder eines mit Holzpartikeln verunreinigten Schmiermittels andererseits gespeichert sind und dass die mit der Kamera (17) aufgenommenen Bilder durch Vergleich mit den gespeicherten Informationen auswertbar sind.

5. Vorrichtung nach Anspruch 3 oder 4, **dadurch gekennzeichnet, dass** die zweite Detektionseinrichtung (D2) eine zusätzliche Lichtquelle aufweist, mit der die zu analysierende Oberfläche des Bandes (4) gleichmäßig ausleuchtbar ist, um gleichbleibende, standardisierende Lichtverhältnisse für die Aufnahme der Bilder mit der Kamera (17) zu schaffen.

6. Vorrichtung nach einem der Ansprüche 1 bis 5, **dadurch gekennzeichnet, dass** die zweite Detektionseinrichtung (D2) als Fluoreszenzmessvorrichtung ausgebildet ist, die zumindest eine elektromagnetische Strahlungsquelle (19) aufweist, mit der elektromagnetische Strahlung eines ersten Wellenlängenbereichs erzeugbar ist und die zumindest einen Sensor (20) aufweist, wobei mit dem Sensor elektromagnetische Strahlung eines zweiten Wellenlängenbereichs, insbesondere Fluoreszenzstrahlung, detektierbar ist, der von dem ersten Wellenlängenbereich abweicht, vorzugsweise oberhalb des ersten Wellenlängenbereichs liegt.

7. Vorrichtung nach einem der Ansprüche 1 bis 6, **dadurch gekennzeichnet, dass** die zweite Detektionseinrichtung (D2) als Interferenzmessvorrichtung ausgebildet ist, die zumindest eine elektromagnetische Strahlungsquelle (19), z. B. eine Röntgenquelle, aufweist und die zumindest einen Sensor (20'), z. B. eine CCD-Kamera, aufweist, wobei mit dem Sensor ein Interferenzmuster der an dem Band (4) und dem auf dem Band (4) befindlichen Material reflektierten und gebeugten Strahlung detektierbar ist.

8. Kontinuierlich arbeitende Presse (1), mit einem oberen endlos umlaufenden Band (4) und einem unteren endlos umlaufenden Band (4),
wobei Pressgut durch den Pressspalt zwischen dem oberen und dem unteren Band (4) hindurchgeführt und unter Anwendung von Druck und/oder Wärme zu einer Platte oder einem endlosen Plattenstrang verpresst wird,
und mit zumindest einer Auftragsvorrichtung (8, 8'), mit der ein Schmiermittel auf das obere Band (4) und/oder auf das untere Band (4) unmittelbar oder mittelbar aufbringbar ist,
**dadurch gekennzeichnet, dass** die Presse (1) mit einer Vorrichtung zur Überwachung des Schmierzustandes nach einem der Ansprüche 1 bis 7 ausgerüstet ist.

9. Verfahren zur Überwachung des Schmierzustandes eines mit einem Schmiermittel beaufschlagten, umlaufenden Bandes (4) für den Transport von Pressgut, insbesondere mit einer Vorrichtung nach einem der Ansprüche 1 bis 7,
wobei mit zumindest einer im Bereich des Bandes (4) angeordneten ersten Detektionseinrichtung (D1) eines ersten Typs und mit zumindest einer im Bereich des Bandes (4) angeordneten zweiten Detektionseinrichtung (D2) eines zweiten Typs jeweils ein Signal erzeugt wird,
wobei mit der ersten Detektionseinrichtung (1) ein von der Menge des auf dem Band befindlichen Materials abhängiges Mengensignal (S1) erzeugt wird,
wobei mit der zweiten Detektionseinrichtung (D2) ein von der Art des auf dem Band befindlichen Materials abhängiges Materialsignal (S2) erzeugt wird, welches davon abhängt, ob sich auf dem Band ein sauberes Schmiermittel oder ein mit Holzpartikeln verschmutztes Schmiermittel oder Holzpartikel befinden, **dadurch gekennzeichnet, dass** die erste Detektionseinrichtung (D1) einen oder mehrere kapazitiv arbeitende Sensoren (9) aufweist, die jeweils zumindest eine in einem Abstand zum Band (4) angeordnete Fühlerplatte (10) aufweisen, die mit dem Band jeweils einen Kondensator (11) bildet, dessen Kapazität von der auf dem Band (4) angeordneten Menge des Materials, nämlich von sauberem Schmiermittel, Holzpartikeln oder mit Holzpartikeln verschmutztem Schmiermittel, abhängt,
und dass aus dem Materialsignal (S2) zwischen einem auf dem Band (4) befindlichen sauberen Schmiermittel einerseits und auf dem Band (4) befindlichen Holzpartikeln oder einem mit Holzpartikeln verunreinigten Schmiermittel andererseits unterschieden wird.

10. Verfahren nach Anspruch 9, dass das Materialsignal (S2) mit einer Kamera (17), und einer Rechnereinheit (18) erzeugt wird, vorzugsweise durch Vergleich von mit der Kamera (17) aufgenommenen Bildern mit in der Rechnereinheit (18) gespeicherten Vergleichsinformationen, z. B. Vergleichsbildern oder Vergleichsmustern.

11. Verfahren zum Betrieb einer Presse nach Anspruch 8, **dadurch gekennzeichnet, dass** der Schmierzustand der Presse (1) mit einem Verfahren nach einem der Ansprüche 9 oder 10 überwacht wird.

## Claims

1. A device for monitoring the lubrication status of a circulating belt (4), to which lubricant is applied, for the transportation of pressing material, more particularly in a continuously operating press (1),
with at least one, when in use, first detection device (D1) of a first type, arranged in the area of the belt (4), and
with at least one, when in use, second detection device (D2) of a second type, arranged in the area of the belt (4),
wherein the first detection device (D1) is configured to produce a quantity signal (S1) as a function of the quantity of the material present on the belt (4),
wherein to differentiate between a lubricant present on the belt (4) and wood particles, or a lubricant contaminated with wood particles present on the belt (4), the second detection device (D2) is configured to produce a material signal (S2) as a function of the type of material present on the belt (4), which is dependent on whether clear lubricant or lubricant contaminated with wood particles or wood particles is/are present on the belt,
**characterised in that** the first detection device (D1) comprises one or more capacitively operating sensors (9), which each have sensor plate (10), which when in use is arranged at a distance from the belt (4) and with the belt forms a capacitor (11) in each case, the capacitance of which depends on the quantity of material, namely clean lubricant, wood particles or lubricant contaminated with wood particles, present on the belt (4).

2. The device according to claim 1, **characterised by** a notification and/or computer system (16) with which the quantity signal (S1) and the material system (S2) can be displayed, and/or with which the quantity signal (S1) and the material signal (S2) can be evaluated in such a way that from the material signal (S2), the type of material present on the belt (4) can be determined so that a distinction can be made between, on the one hand, a clean lubricant present on the belt and, on the other hand, wood particles or a lubricant contaminated with wood particles present on the belt.

3. The device according to claim 1 or 2, **characterised in that** the second detection device (D2) comprises a camera (17) and a computer unit (18), wherein with the camera, one or more images of the material on the belt can be taken, which can be evaluated with the computer unit (18) in such a way that the type of material can be determined and, if applicable, the material signal (S2) can be produced.

4. The device according to claim 3, **characterised in that** in the computer unit (18) of the second detection device (D2), information is stored, e.g. image data, patterns or suchlike of various materials, more particularly of the clean lubricant on the one hand, and, on the other hand, of wood particles and/or a lubricant contaminated with wood particles, and **in that** images taken with the camera (17) can be evaluated through comparison with the stored information.

5. The device according to claim 3 or 4, **characterised in that** the second detection device (D2) comprises an additional light source with which the surface of the belt (4) to be analysed can be evenly illuminated in order to create constant, standardised lighting conditions for taking the images with the camera (17).

6. The device according to any one of claims 1 to 5, **characterised in that** the second detection device (D2) is designed as a fluorescence measuring device, which has at least one electromagnetic radiation source (19) with which electromagnetic radiation of a first wavelength range is can be produced, and which has at least one sensor (20), wherein with the sensor, electromagnetic radiation of a second wavelength range, more particularly fluorescence radiation, is detectable, that differs from the first wavelength range and is preferably above the first wavelength range.

7. The device according to any one of claims 1 to 6, **characterised in that** the second detection devices (D2) is designed as an interference measuring device which has at least one electromagnetic radiation source (19), e.g. an X-ray source, and which has at least one sensor (20'), e.g. a CCD camera, wherein with the sensor, an interference pattern of the radiation reflected and bent on the belt (4) and on the material present on the belt (4) is detectable.

8. A continuously operating press (1) with an upper continuously circulating belt (4), and a lower continuously circulating belt (4),
wherein pressing material is passed through the press gap between the upper and the lower belt (4) and with the use of pressure and/or heat, is pressed to form a board or a continuous board section,
and with at least one application device (8, 8') with which a lubricant can be directly or indirectly applied to the upper belt (4) and/or to the lower belt (4),
**characterised in that** the press (1) is equipped with a device for monitoring the lubrication status in accordance with any one of claims 1 to 7.

9. A method of monitoring the lubrication status of a circulating belt (4), to which lubricant is applied, for the transportation of pressing material, more particularly a device according to any one of claims 1 to 7,
wherein with each of at least one first detection device (D1) of a first type arranged in the area of the belt (4), and with at least one detection unit (D2) of a second type arranged in the area of the belt (4) a signal is produced,
wherein with the first detection device (1), a quantity signal (S1) is produced as a function of the quantity of the material present on the belt,
wherein with the second detection (D2), a material signal (S2) is produced as a function of the type of material present on the belt, which depends on whether a clean lubricant or a lubricant contaminated with wood particles or wood particle is/are present on the belt,
**characterised in that** the first detection device (D1) comprises one or more capacitively operating sensors (9), which each have sensor plate (10), which when in use is arranged at a distance from the belt (4) and with the belt forms a capacitor (11) in each case, the capacitance of which depends on the quantity of the material, namely clean lubricant, wood particles or lubricant contaminated with wood particles, present on the belt (4),
and **in that** from the measuring signal (S2) a distinction can be made between, on the one hand, a clean lubricant present on the belt (4) and, on the other hand, wood particles or a lubricant contaminated with wood particles present on the belt (4).

10. The method according to claim 9, **characterised in that** the material signal (S2) is produced with a camera (17) and a computer unit (18), preferably through comparing images taken by the camera (17) with comparison information, e.g. comparison images or comparison patterns, stored in the computer unit (18).

11. A method of operating a press according to claim 8, **characterised in that** the lubrication status of the press (1) is monitored with a method according to any one of claims 9 or 10.

## Revendications

1. Dispositif de contrôle de l'état de lubrification d'une bande (4) en rotation, alimentée en lubrifiant pour le transport de matériau à presser, en particulier dans une presse fonctionnant en continu (1),
avec au moins un premier système de détection (D1) disposé, en utilisation, dans la zone de la bande (4) d'un premier type et
avec au moins un deuxième système de détection (D2) disposé, en utilisation, dans la zone de la bande (4) d'un deuxième type,
sachant que le premier système de détection (D1) est agencé pour produire un signal de quantité (S1) en fonction de la quantité de matériau se trouvant sur la bande (4),
sachant que pour différencier un lubrifiant se trouvant sur la bande (4) des particules de bois se trouvant sur la bande (4) ou d'un lubrifiant pollué avec des particules de bois, le deuxième système de détection (D2) est agencé pour produire un signal de matériau (S2) en fonction du matériau se trouvant sur la bande (4), lequel est fonction du fait de savoir si un lubrifiant propre ou un lubrifiant encrassé avec des particules de bois ou des particules de bois se trouvent sur la bande,
**caractérisé en ce que** le premier système de détection (D1) comporte un ou plusieurs capteurs (9) fonctionnant de façon capacitive, qui comportent respectivement en utilisation au moins une plaque de détection (10) disposée à une distance de la bande (4), qui forme respectivement avec la bande un condensateur (11), dont la capacité dépend de la quantité de matériau disposée sur la bande (4), notamment de lubrifiant propre, de particules de bois ou de lubrifiant encrassé avec des particules de bois.

2. Dispositif selon la revendication 1, **caractérisé par** un système de signalisation et/ou de calcul (16) avec lequel le signal de quantité (S1) et le signal de matériau (S2) peuvent être affichés et/ou avec lequel le signal de quantité (S1) et le signal de matériau (S2) peuvent être évalués de telle sorte que le type de matériau se trouvant sur la bande peut être déterminé à partir du signal de matériau (S2) de telle manière que l'on peut faire la différence entre un lubrifiant propre se trouvant sur la bande (4) d'une part et des particules de bois ou un lubrifiant pollué avec des particules de bois se trouvant sur la bande d'autre part.

3. Dispositif selon la revendication 1 ou 2, **caractérisé en ce que** le deuxième système de détection (D2) comporte une caméra (17) et une unité de calcul (18), sachant qu'avec la caméra une ou plusieurs images du matériau sur la bande peuvent être prises, lesquelles peuvent être évaluées avec l'unité de calcul (18) de telle sorte que le type de matériau peut être déterminé et le signal de matériau (S2) produit le cas échéant.

4. Dispositif selon la revendication 3, **caractérisé en ce que** des informations sont mémorisées dans l'unité de calcul (18) du deuxième système de détection (D2), par ex. : des données d'images, des modèles ou des informations de même nature de matériaux différents, en particulier du lubrifiant propre d'une part, des particules de bois et/ou d'un lubrifiant pollué avec des particules de bois d'autre part et **en ce que** les images prises avec la caméra (17) peuvent être évaluées par comparaison avec les informations mémorisées.

5. Dispositif selon la revendication 3 ou 4, **caractérisé en ce que** le deuxième système de détection (D2) comporte une source lumineuse supplémentaire avec laquelle la surface de la bande (4) à analyser peut être éclairée de façon uniforme pour créer des conditions de lumière stables, normalisantes pour l'enregistrement des images avec la caméra (17).

6. Dispositif selon l'une quelconque des revendications 1 à 5, **caractérisé en ce que** le deuxième système de détection (D2) est constitué sous la forme d'un dispositif de mesure fluorescent, qui comporte au moins une source de rayonnement électromagnétique (19) avec laquelle un rayonnement électromagnétique d'une première gamme de longueurs d'ondes peut être produit et qui comporte au moins un capteur (20), sachant qu'avec le capteur un rayonnement électromagnétique d'une deuxième gamme de longueurs d'ondes, en particulier un rayonnement fluorescent, peut être détecté, qui dérive de la première gamme de longueurs d'ondes, de préférence au-dessus de la première gamme de longueurs d'ondes.

7. Dispositif selon l'une quelconque des revendications 1 à 6, **caractérisé en ce que** le deuxième système de détection (D2) est constitué sous la forme d'un dispositif de mesure à interférence, qui comporte au moins une source de rayonnement électromagnétique (19), par ex. une source de rayons X, et qui comporte au moins un capteur (20'), par ex. une caméra à dispositif à transfert de charges (*DTC*), sachant qu'avec le capteur, il est possible de détecter un modèle d'interférence du rayonnement diffracté et se réfléchissant sur la bande (4) et sur le matériau se trouvant sur la bande (4).

8. Presse fonctionnant en continu (1) avec une bande (4) supérieure sans fin en rotation et une bande (4) inférieure sans fin en rotation,
sachant que le matériau à presser est passé à travers l'interstice de pressage entre la bande supérieure et la bande inférieure (4) et est comprimé en un panneau ou une ligne de panneaux sans fin en utilisant de la pression et/ou de la chaleur,
et avec au moins un dispositif d'application (8, 8') avec lequel un lubrifiant peut être directement ou indirectement appliqué sur la bande supérieure (4) et/ou sur la bande inférieure (4),
**caractérisé en ce que** la presse (1) est équipée d'un dispositif de contrôle de l'état de lubrification selon l'une quelconque des revendications 1 à 7.

9. Procédé de contrôle de l'état de lubrification d'une bande (4) en rotation alimentée en lubrifiant pour le transport d'un matériau à presser, en particulier avec un dispositif selon l'une quelconque des revendications 1 à 7,
sachant qu'avec au moins un premier système de détection (D1) disposé dans la zone de la bande (4) d'un premier type et avec au moins un deuxième système de détection (D2) disposé dans la zone de la bande (4) d'un deuxième type, un signal est respectivement produit,
sachant qu'avec le premier système de détection (1), un signal de quantité (S1) est produit en fonction de la quantité de matériau se trouvant sur la bande,
sachant qu'avec le deuxième système de détection (D2), un signal de matériel (S2) est produit en fonction du matériau se trouvant sur la bande, lequel dépend du fait qui est de savoir si un lubrifiant propre ou un lubrifiant encrassé avec des particules de bois ou des particules de bois se trouvent sur la bande,
**caractérisé en ce que** le premier système de détection (D1) comporte un ou plusieurs capteurs (9) fonctionnant de façon capacitive, qui comportent respectivement au moins une plaque de détection (10) disposée à une distance de la bande (4), qui forme respectivement avec la bande un condensateur (11), dont la capacité dépend de la quantité de matériau disposée sur la bande (4), notamment de lubrifiant propre, de particules de bois ou de lubrifiant encrassé avec des particules de bois,
et **en ce qu'** à partir du signal de matériau (S2), on peut faire la différence entre un lubrifiant propre se trouvant sur la bande (4) d'une part et des particules de bois ou un lubrifiant pollué avec des particules de bois se trouvant sur la bande (4) d'autre part.

10. Procédé selon la revendication 9, **caractérisé en ce que** le signal de matériau (S2) est produit avec une caméra (17) et une unité de calcul (18), de préférence par comparaison des images prises avec la caméra (17) avec des informations de comparaison mémorisées dans l'unité de calcul (18), par ex. : des images de comparaison ou des modèles de comparaison.

11. Procédé destiné à faire fonctionner une presse selon la revendication 8, **caractérisé en ce que** le niveau de lubrification de la presse (1) est contrôlé avec un procédé selon l'une quelconque des revendications 9 ou 10.
